# FASCICULE DE BREVET EUROPEEN

(11) **EP 0 509 897 B1**
(45) Date de publication et mention de la délivrance du brevet: **01.03.1995**
(21) Numéro de dépôt: 92401034.1
(22) Date de dépôt: 14.04.1992
(51) Int. Cl.: F23G 5/44, B65G 63/04, A61B 19/02

(54) **Installation pour l'incinération de déchets hospitaliers**
Einrichtung zur Verbrennung von Krankenhausabfällen
Installation for incinerating hospital garbage

(30) Priorité: 16.04.1991 FR 9104655
(43) Date de publication de la demande: 21.10.1992
(73) Titulaire: VERI, F-92150 Suresnes (FR); NOVERGIE, F-92150 Suresnes (FR)
(72) Inventeur: Carrese, Michel, F-94160 Saint-Mandé (FR); Riat, Jean-Paul, F-92000 Nanterre (FR)
(74) Mandataire: Dronne, Guy

(56) Documents cités:
- DE-A- 2 222 420
- DE-A- 2 233 435
- DE-B- 1 115 186
- FR-A- 409 488
- GB-A-6010 A.D. 1912 (HEENAN & FROUDE)

## Description

La présente invention concerne une installation pour l'incinération de déchets hospitaliers. Une telle installation est connue du document DE-A-2 233 435.

Les établissements hospitaliers, les cliniques et autres laboratoires d'analyses médicales produisent, de par la nature même de leurs activités, de grandes quantités de déchets, dits déchets hospitaliers. Ces déchets présentent bien sûr une toxicité particulière ou un pouvoir contaminant ou bactériologique particulier qui nécessitent la mise en oeuvre de traitements spécifiques. Actuellement, dans les établissements hospitaliers, les déchets hospitaliers sont recueillis dans un premier temps dans des emballages spéciaux placés à leur tour dans des chariots spécifiques pour être conduits à une installation locale d'incinération. La manipulation de ces déchets impose bien entendu des précautions particulières qui sont relativement lourdes à mettre en oeuvre. En outre, la gestion dans l'hôpital même d'un incinérateur est une opération relativement lourde pour les quantités relativement réduites de déchets hospitaliers à traiter. Leur coût est donc onéreux. En outre, ce traitement des déchets hospitaliers est effectué par le personnel de l'hôpital sans qu'il soit possible de contrôler efficacement que la totalité des déchets sont effectivement incinérés dans des conditions d'hygiène acceptables.

En résumé, les incinérateurs existants actuellement dans les hôpitaux sont, d'une part, d'un coût élevé compte tenu de la quantité limitée de déchets qu'un grand nombre d'hôpitaux ont à traiter. Par ailleurs, le traitement interne de ces déchets ne satisfait pas toujours aux normes qui doivent être appliquées.

Un objet de la présente invention est de fournir une installation pour l'incinération de déchets hospitaliers qui permette d'une part le traitement de quantités importantes de déchets hospitaliers de façon sensiblement continue pour un coût réduit et qui, d'autre part, évite tout risque ou insuffisance dans le traitement de ces déchets, c'est-à-dire que l'installation assure qu'aucun déchet ne sera oublié et donc non traité.

Pour atteindre ce but, l'installation pour l'incinération de déchets hospitaliers dans un four d'incinération, les déchets étant contenus dans des conteneurs mobiles, se caractérise en ce qu'elle comprend un poste de chargement pour solidariser chaque conteneur à des moyens de transfert aérien, lesdits moyens de transfert étant aptes à déplacer automatiquement chaque conteneur de la station de chargement audit four d'incinération, des premiers moyens pour provoquer automatiquement le basculement de chaque conteneur afin de déverser lesdits déchets dans le four, une station de nettoyage et de désinfection desdits conteneurs, lesdits moyens de transfert étant aptes à déplacer automatiquement chaque conteneur dudit four à ladite station de nettoyage, des deuxièmes moyens de basculement pour faire basculer ledit conteneur dans ladite station de nettoyage et pour le ramener dans sa position initiale à la sortie de ladite station de nettoyage et une station de déchargement, lesdits moyens de transfert étant aptes à déplacer ledit conteneur de la station de nettoyage à la station de déchargement.

On comprend qu'une telle installation d'incinération répond effectivement aux impératifs énoncés ci-dessus. D'une part, l'installation étant centrale, elle peut traiter dans des conditions de rendement et de coût intéressants des quantités importantes de déchets hospitaliers provenant d'établissements différents. D'autre part, la manipulation des conteneurs entre le poste de chargement et le poste de déchargement étant entièrement automatisée, le personnel travaille dans des conditions d'hygiène optimales et aucun conteneur ou emballage de déchets hospitaliers contenus dans le conteneur ne peut être omis. En outre, lorsqu'ils retournent à la station de déchargement, les conteneurs ont été nettoyés et désinfectés et leur manipulation ultérieure peut donc être faite sans précaution particulière et sans risque particulier. De préférence, les moyens de transfert aérien sont motorisés.

D'autres caractéristiques et avantages de la présente invention apparaîtront plus clairement à la lecture de la description qui suit d'un mode de réalisation de l'invention donné à titre d'exemple non limitatif. La description se réfère aux figures annexées sur lesquelles :
- la figure 1 est une vue schématique de l'ensemble de l'installation d'incinération ;
- la figure 2a est une vue en élévation des éléments de transfert des conteneurs ;
- la figure 2b est une vue de côté du dispositif de la figure 2a ;
- la figure 3a est une vue longitudinale d'un conteneur de transfert des déchets hospitaliers ;
- la figure 3b est une vue de côté du conteneur de la figure 3a ;
- la figure 4 est une vue en élévation du premier dispositif de basculement pour permettre le déversement des déchets dans le four d'incinération ;
- les figures 5a et 5b sont des vues en élévation respectivement des deuxièmes dispositifs de basculement des conteneurs, ces dispositifs étant situés respectivement à l'entrée et à la sortie de la station de nettoyage et de désinfection ;

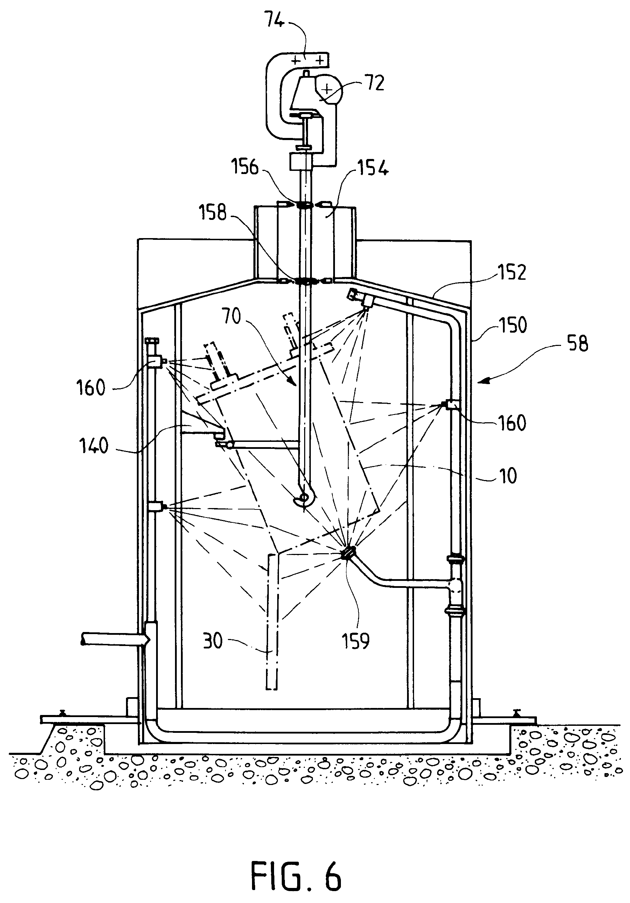

- la figure 6 est une vue en coupe verticale de la station de nettoyage et de désinfection ; et
- les figures 7a, 7b et 7c représentent le conteneur, respectivement dans sa position normale de transfert, dans sa position basculée pour le déversement des déchets dans le four d'incinération et dans sa position basculée pour effectuer l'opération de lavage et de désinfection.

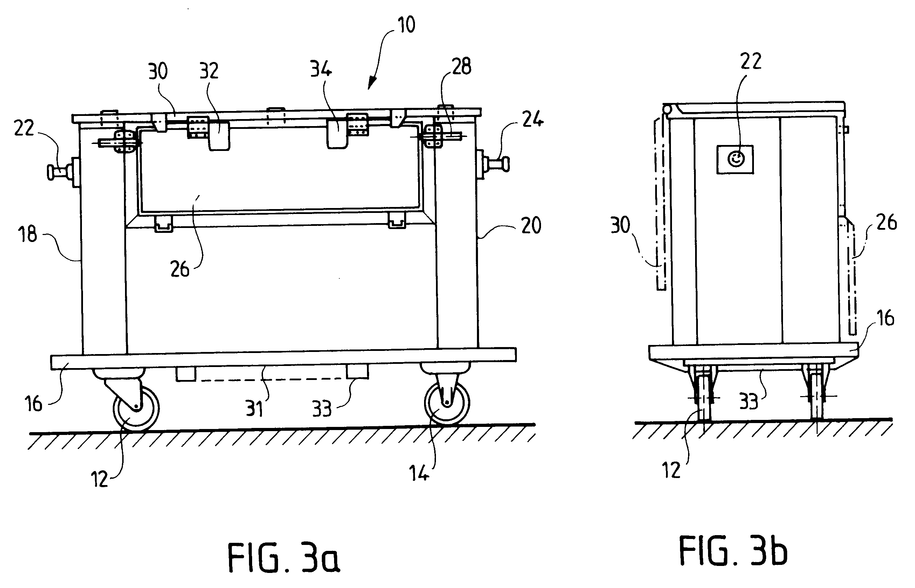

En se référant tout d'abord aux figures 3a et 3b, on va décrire un conteneur utilisé dans l'installation d'incinération. Le conteneur 10 a une forme générale parallélépipédique rectangle montée sur deux roulettes pivotantes 12 et deux roulettes fixes 14. Il se présente donc sous la forme d'un chariot. Le conteneur comporte à sa partie basse un élément de protection mécanique 16 formant pare-choc en saillie par rapport aux flancs du conteneur. Le pare-choc 16 assure non seulement la protection mécanique du conteneur mais il sert également comme moyen d'ancrage pour les opérations de retournement ou basculement, comme on l'expliquera ultérieurement. Sur ses deux faces d'extrémité 18 et 20, le conteneur est muni de tourillons 22 et 24 qui sont excentrés par rapport au plan médian vertical du conteneur. Le conteneur comporte de plus sur une de ses faces latérales une porte basculante 26 à ouverture totale avec un système de verrouillage 28 constitué par des targettes à ressort. La face supérieure du conteneur est constituée par un couvercle 30 à ouverture totale comme le montre mieux la figure 3b. On a représenté sur la figure 3a les systèmes de verrouillage 32 et 34 du couvercle 30.

Lors de son utilisation dans un centre hospitalier, le conteneur est déplacé de façon classique à l'aide des paires de roulettes 12 et 14. Les sacs contenant les déchets hospitaliers sont introduits dans le conteneur par la porte 26 à ouverture totale. Lorsque le conteneur est rempli, la porte 26 est fermée par les targettes 28 et le couvercle est fermé par les éléments de verrouillage 32 et 34. En outre sur son fond 31, le conteneur 10 est muni d'un cadre de manutention au sol 33 (skid en anglais).

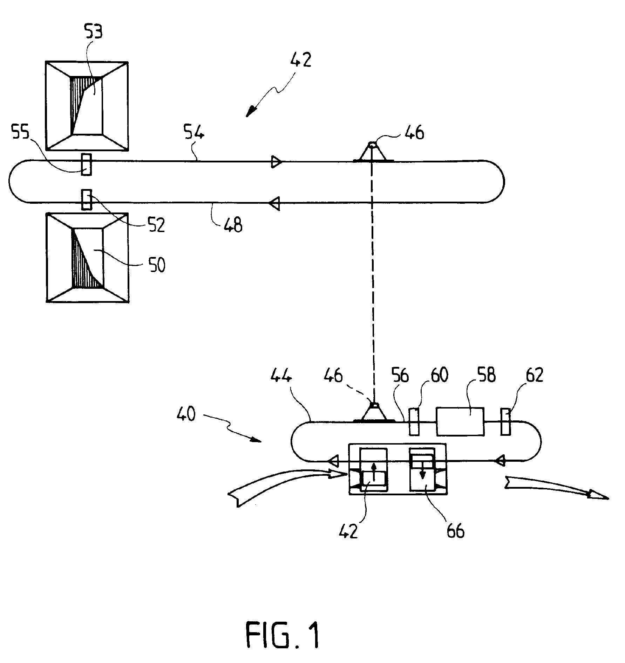

En se référant maintenant à la figure 1, on va décrire l'ensemble de l'installation d'incinération.

L'installation d'incinération occupe le plus souvent deux niveaux, un niveau inférieur portant la référence 40 et un niveau supérieur portant la référence 42. En effet la trémie du four d'incinération est nécessairement disposée à une certaine hauteur correspondant aux dimensions du four, Le niveau inférieur 40 comporte tout d'abord une station 42 de chargement des conteneurs 10. Les conteneurs tels que 10 sont apportés par camion à proximité de la station de chargement 42. Le personnel met en place sur la station 42 chaque conteneur et procède au déverrouillage du couvercle supérieur 30 sans toutefois ouvrir celui-ci. Les conteneurs sont alors pris en charge par un système de transfert par chariot individuel automoteur. Le conteneur pris en charge à la station 42 suit la première portion de son parcours 44, puis il est amené au niveau supérieur 42 par des moyens élévateurs 46. Il suit alors la portion de trajet 48 qui l'amène jusqu'à un four à incinération 50. En regard de ce four à incinération, l'installation comporte une première station de basculement 52 qui permet le basculement de chaque conteneur pour entraîner le transfert des sacs qu'il contient dans la trémie du four 50. Les conteneurs ainsi vidés sont ramenés aux moyens élévateurs 46 qui fonctionnent alors dans le sens de la descente. Ils sont à nouveau pris en charge par le système de convoyage inférieur 40. Ils suivent alors la portion de trajet 56 qui les amène à la station de nettoyage et de désinfection 58. Comme on l'expliquera ultérieurement plus en détails, il est nécessaire de procéder au basculement des conteneurs 10 pour l'opération de nettoyage du fait qu'ils sont étanches. On trouve donc en amont et en aval de la station de nettoyage 58 des dispositifs de basculement respectivement référencés 60 et 62. A la sortie du dispositif de basculement 62, chaque conteneur est repris en charge par les moyens de transfert selon la portion de parcours 64 jusqu'à la station de déchargement 66. A la station de déchargement 66, les conteneurs 10 arrivent nettoyés et désinfectés. Ils peuvent donc être repris en charge par les camions de transport sans précaution particulière, pour être acheminés vers les différents centres hospitaliers ou analogues envoyant leurs déchets hospitaliers dans l'installation qui vient d'être décrite. De plus, grâce à la présence du cadre 33 de manutention au sol prévu sur chaque conteneur 10, celui-ci peut être extrait du camion par un système de manutention au sol qui l'amène à la station de chargement 42. Ce système de manutention au sol est également utilisé entre la station de déchargement et le camion de transport. La manipulation des conteneurs 10 est ainsi totalement automatisée et ne requiert aucune opération manuelle.

En fait, l'installation représentée sur la figure 1 comporte un deuxième four d'incinération 53 associé à une deuxième station de basculement 55. Le deuxième four 53 peut être utilisé en cas de défaillance du premier ou bien les deux fours peuvent fonctionner simultanément si la quantité de déchets à traiter le nécessite.

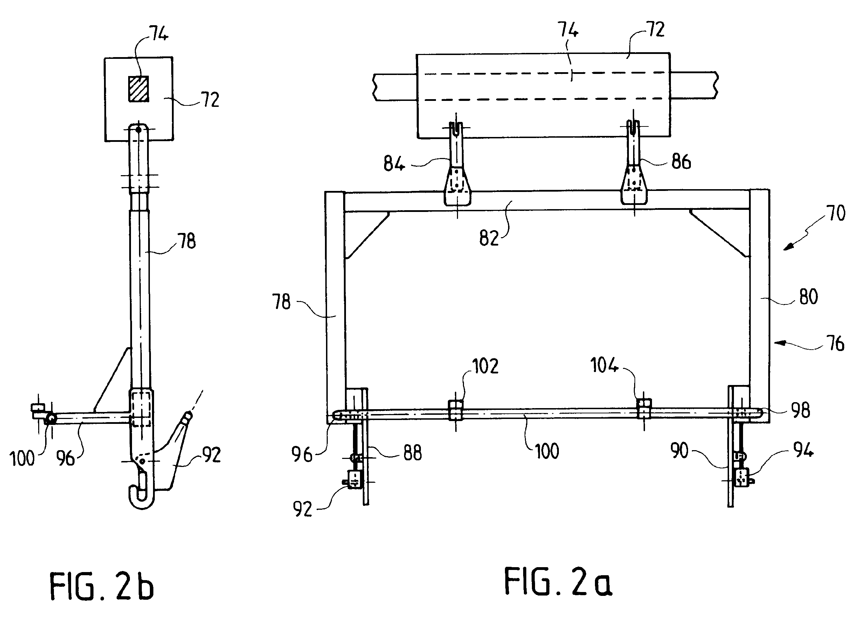

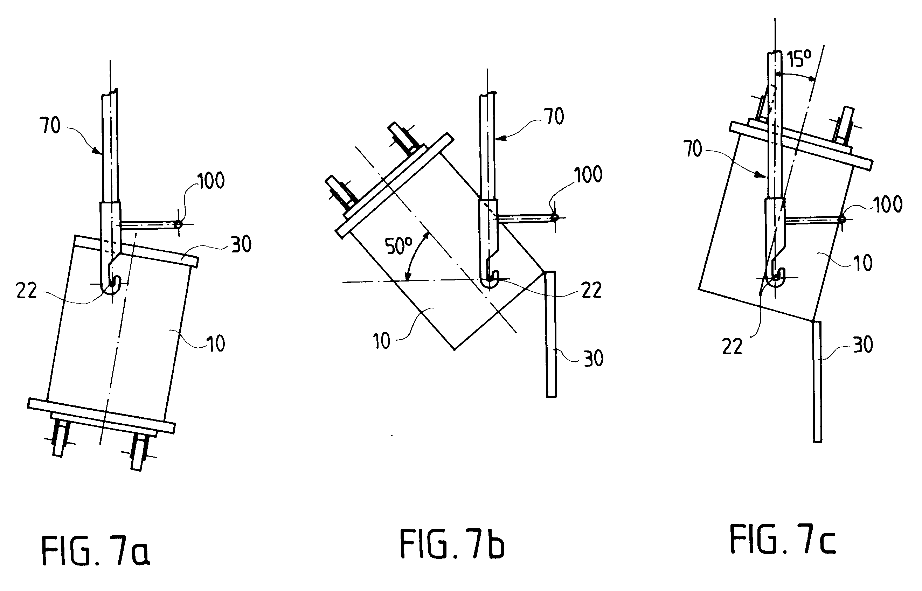

En se référant maintenant aux figures 2a et 2b, on va décrire le mode de transfert automatique des conteneurs 10. Chaque conteneur est déplacé à l'aide d'un chariot de transfert 70 constitué d'une part par des moyens automoteurs 72 coopérant avec un rail de guidage et de supportage 74 et par une balancelle 76. La balancelle 76 comporte deux bras verticaux 78 et 80 fixés aux extrémités d'une traverse horizontale 82, elle-même reliée aux moyens automoteurs 72 par des chapes 84 et 86. Aux extrémités des bras 78 et 80, on trouve des mains de fixation 88 et 90 formant des crochets. Chaque main de fixation est munie d'un linguet, respectivement 92 et 94, à fermeture automatique. On comprend que les crochets 88 et 90 viennent en prise sur les tourillons 22 et 24 de chaque conteneur et que les linguets 92 et 94 assurent la solidarisation temporaire entre le conteneur et chaque chariot de transfert 70. Lors des transferts du conteneur 10, celui-ci occupe la position représentée sur la figure 7a.

La station de déchargement 66 comporte des rampes coopérant avec les linguets 92 et 94 pour permettre leur ouverture et la libération des conteneurs par rapport aux chariots de transfert. Enfin, chaque bras 78 et 80 est muni d'un élément horizontal 96 et 98 à l'extrémité desquels est montée une barre horizontale 100 qui est donc décalée par rapport au plan principal vertical défini par les bras 78 et 80. La barre 100 est munie de deux galets de guidage 102 et 104 dont la fonction sera explicitée ultérieurement.

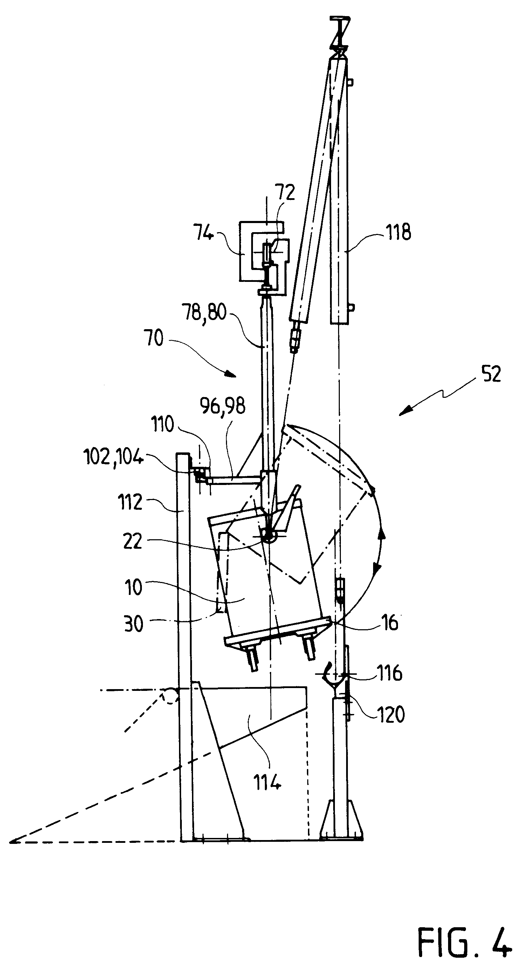

En se référant maintenant à la figure 4, on va décrire un mode préféré de réalisation du dispositif de retournement 52 permettant le déversement du contenu de chaque conteneur 10 dans un four d'incinération.

Lorsqu'il arrive à la station de retournement 52, le conteneur 10 est toujours suspendu au rail de guidage 74 par l'intermédiaire du chariot de transfert 70. Pour stabiliser l'ensemble de supportage, les galets de guidage 102 et 104 sont en prise sur un rail de guidage 110 solidaire d'un châssis 112 enjambant la trémie de la station de retournement. Le conteneur est immobilisé dans une position telle qu'il soit au-dessus de la trémie 114 du four. Le dispositif de basculement est essentiellement constitué par un crochet de préhension 116 actionné par un vérin pendulaire 118. Au repos, le crochet 116 est en position basse et est disposé sur un reposoir 120. Pour provoquer le basculement du conteneur 10, le vérin 118 est commandé pour provoquer un mouvement d'élévation du crochet 116. Celui-ci arrive en prise avec une partie du pare-choc 16 du conteneur 10. Lorsque la remontée du crochet 116 se poursuit, le conteneur 10 amorce un mouvement de pivotement autour de l'axe fixe défini par ses tourillons 22 et 24. En fin de course du vérin, le conteneur présente un angle de vidage de l'ordre de 50°. Comme le couvercle 30 du conteneur 10 avait été préalablement déverouillé, celui-ci s'ouvre spontanément et les déchets hospitaliers tombent par gravité dans la trémie 114.

Lorsque le vidage du conteneur 10 est entièrement réalisé, le vérin 118 est actionné en sens inverse pour provoquer le retour du conteneur 10 dans sa position initiale. Le système d'entraînement automoteur 72 est alors commandé pour provoquer le déplacement du conteneur 10 hors de la station de basculement 52.

La figure 7b montre le conteneur 10 dans sa position de vidage.

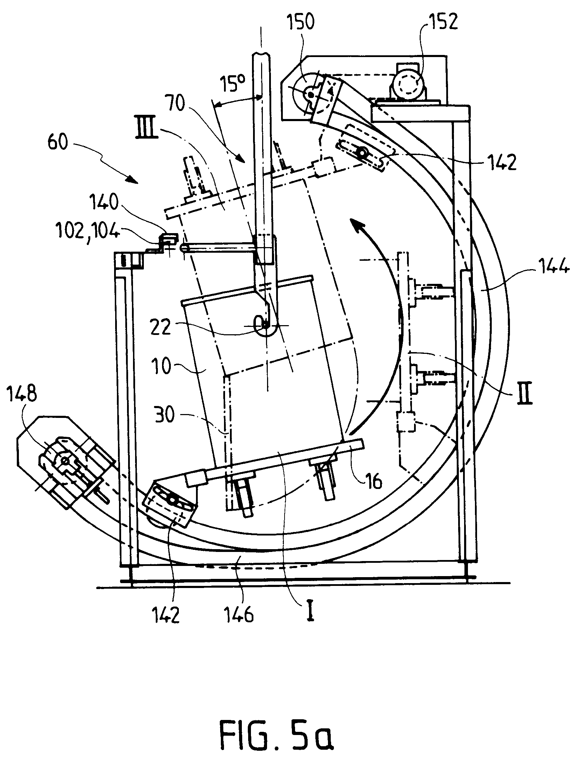

La figure 5a montre un mode préféré de réalisation de la station de retournement 60 disposée à l'entrée de la station de nettoyage et de désinfection 58. Lorsqu'il arrive à la station de retournement 60 le conteneur 10 est suspendu à l'ensemble de transfert 70. En outre, les galets 102 et 104 sont engagés dans un rail de guidage 140. Ainsi, l'ensemble de transfert 70 est immobilisé en position verticale. Le dispositif de retournement comporte essentiellement un poussoir 142 apte à prendre en charge le conteneur 10 par une partie de son pare-choc 16. Le poussoir 142 peut se déplacer sur un rail de guidage en forme de demi-cercle référencé 144. Le poussoir 142 est déplacé à l'aide d'une chaine bouclée à galets 146. La chaine passe d'une part sur un galet tendeur 148 et d'autre part sur un galet d'entraînement 150. Le galet d'entraînement 150 est relié à un groupe moto-réducteur 152. Sur la figure 5a, on a représenté le conteneur 30 dans sa position initiale I, dans une position intermédiaire II et dans sa position finale III. Dans cette dernière position, le conteneur est en appui sur la barre horizontale 100 solidaire du chariot de transfert 70. Comme le montre mieux la figure 7c, dans sa position retournée de lavage, le conteneur 30 a son plan médian qui fait un angle de 15° avec la verticale.

Le conteneur 10 est introduit dans cette position à l'intérieur de la station de nettoyage et de désinfection 58. L'enceinte 150 de la station de nettoyage comporte un toit 152 qui est fendu. Plus précisément, il est constitué par une gaine fendue 154 maintenue en surpression. L'étanchéité est assurée par un double rideau 156 et 158 de poils en matière plastique. A l'intérieur de l'enceinte 150, on trouve d'une part une buse rotative 159 qui permet de nettoyer et de désinfecter l'ensemble de la partie interne du conteneur 10 grâce à l'inclinaison qui lui a été donnée au préalable et trois rampes de quatre buses latérales fixes telles que 160 permettant d'effectuer le nettoyage et la désinfection de la totalité des parois externes du conteneur 10. Les extrémités verticales de l'enceinte 150 sont constituées par des portes d'entrée et de sortie, non représentés, isolant l'extérieur de toute projection d'eau polluée. Lors du rinçage, l'eau est additionnée d'un produit désinfectant dont la teneur est parfaitement contrôlée. Durant tout le séjour du conteneur dans l'enceinte 150, les galets 102, 104 restent engagés dans un rail de guidage prolongeant le rail 140 de la station de retournement 60.

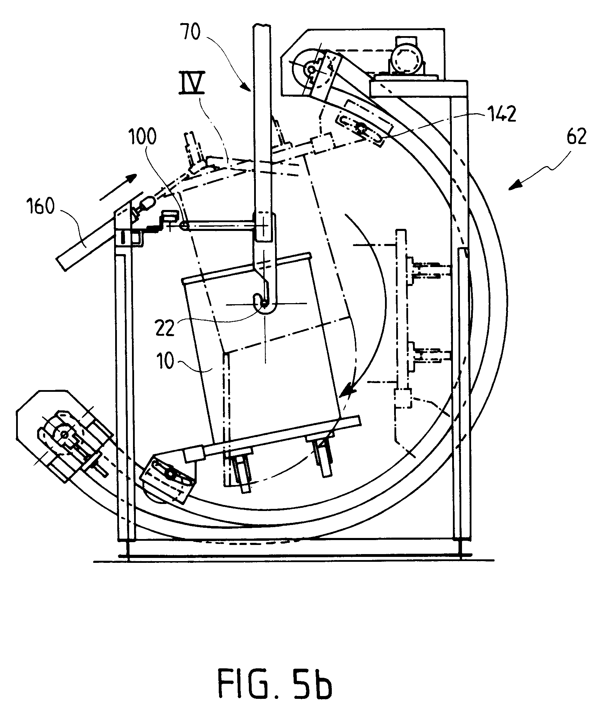

A sa sortie de la station de nettoyage 58, le conteneur 10 passe dans une station de basculement 62 pour ramener le conteneur 10 dans sa position initiale, c'est-à-dire sa position normale de transfert. La station de basculement 62 est identique à la station de basculement 60 à l'exception du fait que celle-ci comporte de plus un vérin 160 pour faire passer le conteneur de sa position où il est en appui sur la barre 100 solidaire du dispositif de supportage 70 à une position modifiée repérée par la référence IV sur la figure 5b. Dans cette position, le conteneur 10 est repris en charge par le poussoir 142 qui le ramène dans sa position de transfert.

Le chariot automoteur de transfert 70 ramène alors le conteneur 10 à la station de déchargement 66. Le conteneur 10 est alors libéré du chariot de transfert 70. Il peut être récupéré par le personnel chargé du transfert des conteneurs et installé dans le véhicule qui le ramènera vers un centre hospitalier.

Il va de soi que le système de transfert aérien des conteneurs 10 pourrait ne pas être motorisé avec des chariots commandés indépendamment. Les balancelles 70 pourraient faire partie d'une chaîne de transfert mono ou bi-rail.

Selon un mode de mise en oeuvre perfectionné, chaque conteneur 10 comporte une étiquette externe identifiant la provenance du conteneur. Cette étiquette est lue automatiquement à la station de chargement 42. De plus cette dernière comporte une installation de pesage de conteneurs. Cette information de poids de déchets à traiter permet d'optimiser la gestion du chargement du ou des fours d'incinération.

De plus, on dispose pour chaque conteneur de l'information de provenance et de l'information de poids de déchets hospitaliers contenus. Ces deux informations permettent d'établir le montant de la facture à transmettre au centre hospitalier qui a envoyé les déchets.

Il découle de la description précédente que l'installation d'incinération selon l'invention présente de nombreux avantages par rapport à celles de l'état de la technique. Outre les avantages déjà mentionnés, il faut souligner que le transfert des conteneurs est effectué à l'aide de moyens auto-moteurs, ce qui permet de réduire les coûts et de donner à l'installation une beaucoup plus grande souplesse d'exploitation en fonction des quantités de déchets hospitaliers à traiter.

Enfin les conteneurs 10 passent nécessairement par la station de nettoyage et de désinfection avant de parvenir à la station de déchargement. On est ainsi certain que la manipulation manuelle des conteneurs ne porte que sur des conteneurs désinfectés.

## Revendications

1. Installation pour l'incinération de déchets hospitaliers dans un four d'incinération, lesdits déchets étant contenus dans des conteneurs, caractérisée en ce qu'elle comprend :
- un poste de chargement (42) pour solidariser un conteneur (10) à des moyens de transfert aérien (70), lesdits moyens de transfert étant aptes à déplacer automatiquement un conteneur (10) de la station de chargement audit four d'incinération (50), des premiers moyens (52) pour provoquer automatiquement le basculement dudit conteneur pour déverser lesdits déchets dans ledit four, une station de nettoyage et de désinfection (58) desdits conteneurs, lesdits moyens de transfert étant aptes à déplacer automatiquement ledit conteneur dudit four (50) à ladite station de nettoyage (58), des deuxièmes moyens de basculement (60, 62) pour faire basculer ledit conteneur dans ladite station et pour le ramener dans sa position initiale à la sortie de ladite station, et une station de déchargement, lesdits moyens de transfert étant aptes à déplacer ledit conteneur (10) de ladite station de nettoyage à ladite station de déchargement (66).

2. Installation selon la revendication 1, caractérisée en ce que lesdits conteneurs (10) ont la forme de chariots à roulettes (12, 14), en ce qu'ils comportent un couvercle supérieur (30) à ouverture totale, et en ce qu'ils sont munis de deux tourillons (22, 24) pour leur solidarisation sur lesdits moyens de transfert (70).

3. Installation selon la revendication 2, caractérisée en ce que lesdits moyens de transfert comprennent un chariot (72) apte à coopérer avec un rail de guidage et de supportage (74) et une balancelle (76) comportant deux mains (88, 90) de préhension des tourillons (22, 24) des conteneurs (10) et une barre horizontale (100) solidaire de ladite balancelle (76) et décalée par rapport au plan vertical de ladite balancelle.

4. Installation selon l'une quelconque des revendications 2 et 3, caractérisée en ce que ledit conteneur (10) comprend en outre des moyens de saisie (16) proches de l'extrémité inférieure dudit conteneur et disposés perpendiculairement à l'axe défini par lesdits tourillons (22, 24) et en ce que lesdits premiers et deuxièmes moyens de basculement (52, 60) comprennent des moyens (110, 140) aptes à coopérer avec des moyens de guidage (102, 104) solidaires de ladite balancelle (76) pour immobiliser verticalement lesdits moyens de transfert (70) et des moyens de déplacement (116, 118 et 142) aptes à coopérer avec lesdits moyens de saisie (16) du conteneur pour provoquer un pivotement de celui-ci autour de l'axe défini par ses tourillons (22, 24).

5. Installation selon la revendication 4, caractérisée en ce que lesdits moyens de déplacement (142) desdits deuxièmes moyens de basculement (60) sont aptes à amener un côté dudit conteneur (10) en appui stable sur ladite barre horizontale (100) de ladite balancelle (76).

6. Installation selon l'une quelconque des revendications 1 à 5, caractérisée en ce que ladite station de nettoyage et de désinfection (58) comprend une enceinte (150) fermée à sa partie supérieure (52) par des moyens (154, 156) autorisant le passage desdits moyens de transfert (70) de façon quasi-étanche.

7. Installation selon l'une quelconque des revendications 1 à 6, caractérisé en ce que ledit conteneur (10) traverse ladite station de nettoyage (58) dans sa position basculée, et en ce que ladite station de nettoyage (58) comprend des buses d'injection de liquide (159, 160) pour diriger des jets de liquide vers les parois internes du conteneur (10) et vers ses parois externes.

8. Installation selon l'une quelconque des revendications 1 à 7, caractérisée en ce que lesdits moyens de transfert aérien (70) sont automoteurs.

## Patentansprüche

1. Anlage zum Verbrennen von in Behältern enthaltenen Krankenhausabfällen in einem Verbrennungsofen,
dadurch gekennzeichnet, daß sie umfaßt:
- eine Ladestelle (42), um einen Behälter (10) mit Überflur-Überführungsmitteln (70) fest zu verbinden, wobei diese Überführungsmittel geeignet sind, einen Behälter (10) selbsttätig von der Ladestation zum Verbrennungsofen (50) zu versetzen, erste Mittel (52) zum selbstättigen Kippen des Behälters, um die Abfälle in den Ofen auszukippen, eine Reinigungs- und Desinfektionsstation (58) für die Behälter, wobei die Überführungsmittel geeignet sind, den Behälter selbsttätig vom Ofen (50) zur Reinigungssation (58) zu versetzen, zweite Mittel zum Kippen (60,62), um den Behälter in dieser Station zu kippen und um ihn am Ausgang dieser Station in seine Anfangsstellung zurückzuführen, und eine Ausladestation, wobei diese Überführungsmittel geeignet sind, den Behälter (10) von der Reinigungsstation zur Ausladestation (66) zu versetzen.

2. Anlage nach Anspruch 1,
dadurch gekennzeichent, daß die Behälter (10) die Gestalt von Wagen mit Rollen (12,14) haben, dadurch, daß sie einen vollständig öffenbaren oberen Deckel (30) aufweisen, und dadurch, daß sie mit zwei Zapfen (22,24) ausgestattet sind, um sie mit den Überführungsmitteln (70) fest zu verbinden.

3. Anlage nach Anspruch 2,
dadurch gekennzeichnet, daß die Überführungsmittel einen Wagen (72) aufweisen, der geeignet ist, mit einer Führungs- und Trageschiene (74) zusammenzuwirken, und ein Gehänge (76) mit zwei Greifhänden (88,90) für die Zapfen (22,24) der Behälter (10), und eine waagerechte Stange (100), die an dem Gehänge (76) befestigt und gegenüber der senkrechten Ebene des Gehänges versetzt angeordnet ist.

4. Anlage nach Anspruch 2 oder 3,
dadurch gekennzeichnet, daß der Behälter (10) weiterhin Eingriffsmittel (16) aufweist, die dem unteren Ende des Behälters benachbart und senkrecht zu der durch die Zapfen (22,24) festgelegten Achse angeordnet sind, und dadurch, daß die ersten und zweiten Mittel zum Kippen (52,60) Mittel (110,140) aufweisen, die geeignet sind, mit an dem Gehänge (76) befestigten Führungen (102,104) zusammenzuwirken, um die Überführungsmittel (70) in senkrechter Richtung festzuhalten, und Verschwenkmittel (116,118 und 142), die geeignet sind, mit den Eingriffsmitteln (16) des Behälters zusammenzuwirken, um diesen um die durch seine Zapfen (22,24) festgelegte Achse zu schwenken.

5. Anlage nach Anspruch 4,
dadurch gekennzeichnet, daß die Verschwenkmittel (142) der zweiten Mittel zum Kippen (60) dazu geeignet sind, eine Seite des Behälters (10) in eine stabile Auflage auf der waagerechten Stange (100) des Gehänges (76) zu bringen.

6. Anlage nach einem der Ansprüche 1 bis 5,
dadurch gekennzeichent, daß die Reinigungs- und Desinfektionsstation (58) einen umschlossenen Raum (150) aufweist, der an seinem oberen Abschnitt (52) mit Mitteln (154,156) geschlossen ist, die den Durchtritt der Überführungsmittel (70) in einer nahezu hermetischen Weise erlauben.

7. Anlage nach einem der Ansprüche 1 bis 6,
dadurch gekennzeichnet, daß der Behälter (10) die Reinigungsstation (58) in seiner gekippten Stellung durchläuft, und dadurch, daß die Reinigungsstation (58) Flüssigkeits-Einspritzdüsen (159,160) aufweist, um Flüssigkeitsstrahlen gegen die inneren Wandungen des Behälters (10) und gegen seine äußeren Wandungen zu richten.

8. Anlage nach einem der Ansprüche 1 bis 7,
dadurch gekennzeichnet, daß die Überflur-Überführungsmittel (70) mit Eigenantrieb ausgestattet sind.

## Claims

1. Installation for the incineration of hospital wastes in an incineration furnace, said wastes being contained in containers, characterised in that it includes:
- a loading station (42) for rigidly connecting a container (10) to aerial transfer means (70), said transfer means being capable of automatically displacing a container (10) from the loading station to said incineration furnace (50), first means (52) for automatically causing tilting of said container in order to pour said wastes into said furnace, a station (58) for cleaning and disinfecting said containers, said transfer means being capable of automatically displacing said container from said furnace (50) to said cleaning station (58), second tilt means (60, 62) for tilting said container in said station and for returning it to its initial position at the exit of said station, and an unloading station, said transfer means being capable of displacing said container (10) from said cleaning station to said unloading station (66).

2. Installation according to claim 1, characterised in that said containers (10) are in the form of carriages with wheels (12, 14), in that they comprise a fully-opening upper cover (30), and in that they are provided with two journals (22, 24) for rigid connection thereof to said transfer means (70).

3. Installation according to claim 2, characterised in that said transfer means include a carriage (72) capable of cooperating with a guiding and supporting rail (74) and a swing tray (76) comprising two arms (88, 90) for gripping the journals (22, 24) of the containers (10) and a horizontal bar (100) rigidly connected to said swing tray (76) and offset from the vertical plane of said swing tray.

4. Installation according to either of claims 2 and 3, characterised in that said container (10) further includes gripping means (16) close to the lower end of said container and arranged perpendicularly to the axis defined by said journals (22, 24) and in that said first and second tilt means (52, 60) include means (110, 140) capable of cooperating with guiding means (102, 104) rigidly connected to said swing tray (76) for vertically locking said transfer means (70) and displacement means (116, 118 and 142) capable of cooperating with said gripping means (16) of the container in order to cause pivoting thereof about the axis defined by its journals (22, 24).

5. Installation according to claim 4, characterised in that said displacement means (142) of said second tilt means (60) are capable of bringing one side of said container (10) into stable abutment against said horizontal bar (100) of said swing tray (76).

6. Installation according to any of claims 1 to 5, characterised in that said cleaning and disinfecting station (58) includes an enclosure (150) closed in its upper region (52) by means (154, 156) allowing passage of said transfer means (70) in an almost sealing-tight manner.

7. Installation according to any of claims 1 to 6, characterised in that said container (10) passes through said cleaning station (58) in its tilted position, and in that said cleaning station (58) includes liquid injection nozzles (159, 160) for directing jets of liquid towards the inner walls of the container (10) and towards its outer walls.

8. Installation according to any of claims 1 to 7, characterised in that said aerial transfer means (70) are self-propelled.
